Europäisches Patentamt

(19) European Patent Office

Office européen des brevets

(11) Publication number: **0 335 726**
**A2**

(12) **EUROPEAN PATENT APPLICATION**

(21) Application number: **89303179.9**

(51) Int. Cl.⁴: **A 61 K 37/02**

(22) Date of filing: **30.03.89**

(30) Priority: **31.03.88 US 175912**

(43) Date of publication of application:
**04.10.89 Bulletin 89/40**

(84) Designated Contracting States:
**BE CH DE ES FR GB IT LI**

(71) Applicant: **IMMUNOBIOLOGY RESEARCH INSTITUTE, INC.**
**Route 22 East P.O. Box 999**
**Annandale New Jersey 08801-0999 (US)**

(72) Inventor: **Goldstein, Gideon**
**30 Dorison Drive**
**Short Hills New Jersey 07078 (US)**

**Hirsch, Robert**
**20 Valinor Road**
**Belle Mead New Jersey 08502 (US)**

**Meyerson, Linda**
**3645 Starr Route**
**Aquetong Road Carversville, PA 18913 (US)**

(74) Representative: **Hale, Stephen Geoffrey et al**
**J.Y. & G.W. Johnson Furnival House 14/18 High Holborn**
**London WC1V 6DE (GB)**

The title of the invention has been amended (Guidelines for Examination in the EPO, A-III, 7.3).

(54) **Use of thymopentin and derivatives in treatment of HIV viremic patients.**

㊗ The viremia of HIV in the body fluids of patients having peripheral blood mononuclear cells capable of transmitting HIV virus to non-infected cells upon co-culture is reduced by administration of a pharmaceutical composition containing the pentapeptide thymopentin or an analog or derivative thereof.

EP 0 335 726 A2

**Description**

# TREATMENT OF HIV VIREMIC PATIENTS

The present invention relates generally to means for the treatment of a class of HIV infective patients. More particularly, the invention involves means for use in the control of HIV viremia in the body fluids of patients in whom infective HIV is being released by peripheral blood mononuclear cells, as detected by co-culture.

Thymopentin is a synthetic pentapeptide, having the sequence H-Arg-Lys-Asp-Val-Tyr-OH, corresponding to the active site of the thymic hormone thymopoietin. [See, e.g. Goldstein et al, Science, 204:1309-1310 (1979) and US-A-4,190,646]. Thymopentin has been developed for pharmaceutical administration as a parenteral drug, and has proved valuable in treating certain chronic infections characterized by an immunosuppressed state of the host. Conditions for which thymopentin has been employed therapeutically include lepromatous leprosy and severe recurrent herpes virus infections. This pentapeptide has also been employed in the treatment of certain allergies. Thymopentin has proved to be an outstandingly safe therapeutic agent in both a wide range of animal studies and extensive clinical trials for these and other conditions. [See, e.g. E. Sundal et al, "Therapy with thymopentin: A clinical overview", in Immune Regulation by Characterized Peptides, eds: Goldstein et al, Alan R. Liss, Inc. NY, pp 121-136 (1987); A. Castells et al, Surv. Immunol. Res., 4:63-69, Suppl. 1 (1985); J. Demaubeuge et al, Surv. Immunol. Res., 4:30-36, Suppl. 1 (1985); copending U.S. Patent Application Ser. No. 822,704; N Friedmann, Surv. Immunol. Res., 4:139-148, Suppl. 1 (1985); and P. Weiss et al, Surv. Immunol. Res., 4:149-154 Suppl.1 (1985)].

Thymopentin has also been employed in experimental therapy with patients having acquired immunodeficiency syndrome, or AIDS, a disease predominantly caused by transmission of the retrovirus HIV-1, and with a category of patients demonstrating symptoms of AIDS related complex, or ARC. [see e.g. N. Clumeck et al, Int. J. Clin. Pharm. Res., 4:459-463 (1984); W. Barcellini et al, Clin. Exp. Immunol., 67:537-543 (1987); N. Clumeck et al, Surv. Immunol. Res., 4:58-62 Suppl. 1 (1985); F. Mascart-Lemone et al, Lancet, 2:735-736 (1983); and A. Schedel et al, Akt. Dermatol., 11:187-189 (1985)]. All such patients are characterized by one or more of the following symptons: opportunistic infection, persistent generalized lymphadenopathy, dementia, fever, weight loss, diarrhea, hepatosplenomegaly. However, not all such patients are viremic. The results obtained in these reports were ambiguous, ranging from reports of no effect on full-blown AIDS patients to some improvement in the immunological profile indices of ARC patients, e.g. responses to certain mitogens.

Presently employed therapeutic approaches for the treatment of AIDS have proved of limited value to date, including treatment with antiviral agents, such as zidovudine, and immunoregulators, such as ampligen. Attempts at vaccine developments have so far been unsuccessful. Additionally, no agent to date has been demonstrated effectively to control or reduce the infectivity of the virus once a patient is infected. The infectivity of HIV virus through contact with body fluids is a crucial concern because contact by uninfected persons with body fluids of viremic subjects spreads the disease. Such contact primarily involves transmission of the virus through blood and semen during sexual intercourse, both homosexual and heterosexual; through blood by the use of "dirty" needles by intravenous drug addicts; through tainted blood products, e.g. serum-derived drugs, such as Factor VIII for hemophiliacs, or recipients or donor blood infected with HIV. In addition to such medical concerns, the infectivity of body fluids of HIV viremic patients has created a number of social concerns about transmission of the disease, e.g. to uninfected persons engaged in the hospitalization and treatment of HIV infected patients as well as to uninfected persons exposed to persons infected with the HIV virus who are sufficiently healthy to continue "normal" activities, such as attendance at schools and places of employment.

Therefore, there remains a need in the art for effective treatments to stem the infectivity of the HIV virus and the resulting decimation of human health and lives caused thereby.

The present invention surprisingly provides, in view of equivocal results of previous studies of thymopentin in treatment of AIDS and ARC patients, means for treating a specific class of HIV infected patients to reduce the infectivity of the virus in their body fluids, particularly blood.

The invention provides the use of an active ingredient selected from thymopentin and analogs and derivatives thereof for preparation of a pharmaceutical composition suitable for reducing the infectivity of HIV virus in the peripheral blood cells and other body fluids of a viremic subject having peripheral blood mononuclear cells capable of transmitting the HIV virus to uninfected cells in co-culture.

The resulting pharmaceutical composition, containing an effective amount of the active ingredient, preferably thymopentin or a pharmaceutically-acceptable salt thereof, enables the infectivity of HIV virus in viremic patients, i.e. patients having peripheral blood mononuclear cells (PBM) capable of transmitting the infection to uninfected normal cells in co-culture, to be reduced when it is administered to such patients for a time sufficient to reduce viremia in the blood and other body fluids to low levels or to completely eliminate viremia in such fluids. The reduction of viremia which is the result of the method may be ascertained by repeating conventional co-culture protocols of the patients' PBMs together with those of an uninfected normal subject.

In a double-blind study, thymopentin treatment of viremic, asymptomatic patients in whom infective HIV-1 was being released by PBM, as detected by co-culture, was found to reduce the viremia (virus replication and release) in blood in a statistically significant manner compared to placebo, presumably by stimulating the host-defense mechanisms controlling viral replication.

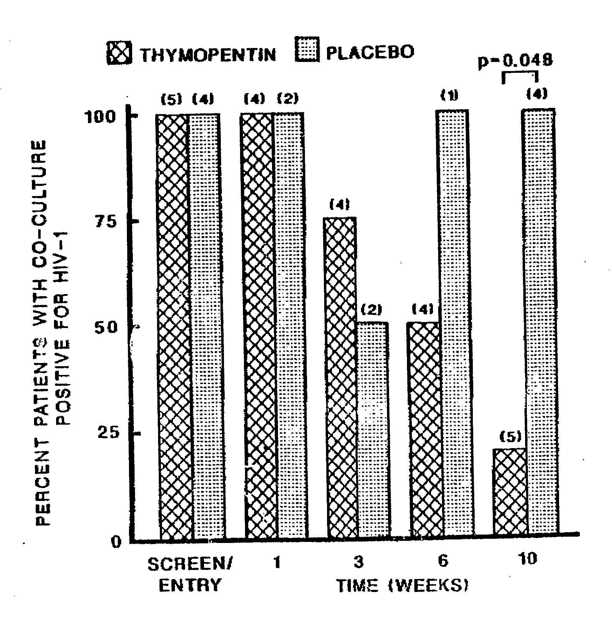

The sole figure of the accompanying drawing is a bar graph showing the percentage of patients along the ordinate positive for HIV-1 infectivity in co-culture when treated with thymopentin (cross-hatched bars) or a placebo (striped bars) at testing intervals (along the abscissa) of one, three, six and 10 weeks. N indicates the number of subjects tested in that group for each time point.

By means of the present invention the replication and infectivity of the HIV virus can be reduced in those patients having peripheral blood mononuclear cells which, upon co-culture, are capable of infecting non-infected PBM with the virus by a method involving administering to those patients an effective amount of the pentapeptide thymopentin (or of an analog or derivative thereof having a similar effect) capable of progressively reducing the HIV infectivity of the patients' PBM, as detected by conventional co-culture techniques. Yet another class of patients at whom this therapeutic method is directed are those patients whose PBMs are capable of transmitting the HIV virus, yet who display no symptomatic indications of AIDS or ARC infection.

The therapeutic method preferably involves a two-stage treatment protocol. Viremic patients, whether symptomatic or asymptomatic, are treated with thymopentin in an amount effective to substantially reduce the level of viremia or preferably to reduce the infectivity of PBMs to an undetectable level in co-culture with non-infected cells. Upon determination of acceptably low levels of infectivity or, preferably, elimination of viremia, the regimen and amounts of thymopentin administration are adjusted to enable maintenance of this substantially non-viremic state.

The thymopentin administered according to this method may be prepared as described in US-A-4,190,646, which patent is incorporated by reference for its disclosure of the preparation, formulations and background of thymopentin. Pharmaceutically acceptable derivatives such as salts of thymopentin, e.g. as disclosed in the above-mentioned patent, may also be used. It is also expected that other analogs of thymopentin may be employed in the present invention. Such analogs include those described in US-A-4,261,886; 4,397,842; 4,505,853 and 4,629,723; which patents are also incorporated by reference. Pharmaceutical formulations containing thymopentin, an analog or pharmaceutically acceptable derivative (such as a salt) thereof may be used in this method, including ingredients such as conventional pharmaceutically acceptable carriers, like buffered saline or sterile water, or conventional ingredients to aid solubility or preservation of the formulation. For convenience, however, the active ingredient will hereafter be referred to as thymopentin.

The preferred mode of administration of thymopentin to achieve the desired reduction in HIV infectivity is parenteral, more preferably intravenous or subcutaneous, and most preferably subcutaneous. Where desirable, other parenteral routes may be employed to deliver the effective amount of thymopentin, including percutaneous, intramuscular, buccal, intranasal, and vaginal or rectal (via suppositories). The pharmaceutical compositions used for administration may contain, for example, from about 5 mg to about 500 mg thymopentin, preferably in the region of 50 mg thymopentin. Certain of the thymopentin analogs referred to above have been developed for oral administration. Where such analogs are employed in the method of the invention, oral administration may be preferred. It is not believed, however, that the specific mode of administration is critical to the practice of the present method so long as an effective amount of thymopentin enters the blood stream.

For the first stage of thymopentin administration to reduce or eliminate viremia in the body fluids of subjects whose PBM's have been determined to be capable of transmitting the virus to non-infected cells, it is expected that a dosage of from 25 mg to about 500 mg thymopentin is acceptable. This dosage could be administered daily or several times per week for one or more months until an acceptably low or non-viremic stage is reached, based on co-culture results. We have found that 50 mg of thymopentin administered subcutaneously three times per week was an effective regimen to reduce viremia in asymptomatic AIDS patients, as shown in the study described in the Example below and in the drawing. However, it is believed to be well within the skill of a practitioner in the field to determine other appropriate doses of thymopentin and frequencies of administration to achieve the desired reduction of viremia. For subcutaneous administration, those skilled in the art would recognize the necessity to increase the dose. Since thymopentin is a very safe drug, the only practical upper limit is dictated by optimum efficacy. The pharmaceutical compositions may for example be in unit dosage form containing sufficient thymopentin for daily administration or for administration 1 - 3 times per week.

Once a stage of low or non-viremia is achieved, the patient's therapy, including amount, route of administra tion and/or frequency of administration, may be altered to maintain the non-viremic state. For example, such "maintenance" therapies may include administration of 50 mg thymopentin subcutaneously less than three times a week. It is considered that a maintenance dosage may be delivered once a week or even bi-weekly or less frequently, depending on the periodic co-culture assessments of non-viremia, and the pharmaceutical compositions for use in this period may conveniently contain the maintenance amount of thymopentin. Such treatment may be expected to continue indefinitely to maintain a non-viremic state in such patients. All such doses, frequencies of administration, and modes of administration are intended to be included.

Efficacy of this method is determined by periodic performance of a conventional co-culture assay which measures the initial HIV infectivity of PBM and also the resulting reduction thereof upon treatment with thymopentin. Such a co-culture method is described in L. A. Evans, J. Immunol., 138:3415-3418 (1987). Other similar techniques for evaluating the infectivity of the virus in body fluids of an HIV infected patient may also be employed.

The following Example demonstrates the method in treating viremic and asymptomatic HIV-1 infective patients with thymopentin to reduce the infectivity of the virus in body fluids, such as blood.

### EXAMPLE

Asymptomatic patients from known risk groups were screened for antibodies to HIV-1 by standard clinical laboratory enzyme-linked immunoassay (ELISA), with confirmation by Western blot. Sixty-four HIV-1 antibody positive patients were further screened for HIV-1 infectivity of PBM by co-culture of their phytohemagglutinin (PHA) activated PBM with PHA activated PBM from normal subjects or, alternatively, HUT 78 cells, a human cell line, according to procedures described in L. A. Evans, J. Immunol., supra. Because HUT 78 cells proved much less sensitive than PBM for HIV-1 detection, all testing was eventually performed by co-culture with PBM. Culture supernatants were assayed for the P24 antigen of HIV-1 by ELISA according to procedures described in the brochure accompanying the ELISA assay kit [duPont Biotechnology Systems, NEN Research Products, HIV(HTLV-III/LAV) p. 24, ELISA System, Catalog #NEK-041 NEK-041A, Package insert], to determine if the patient's PBM were producing virus capable of infecting the co-cultured cells. Of the 64 antibody positive patients, fifteen (23%) proved positive (i.e. had PBM which produced HIV-1 virus capable of infecting the non-infected PBM) and were admitted to the study.

Nine patients with PBM positive for HIV-1 on co-culture were randomly assigned on a double-blind basis to treatment with either 50 mg thymopentin (5 patients) or a placebo (4 patients) subcutaneously three times per week for six weeks. In this example, thymopentin was prepared as described in US-A-4,190,646 and administered in a carrier of buffered saline. PBM of the treated subjects were cultured for HIV-1 at one, three, six and ten weeks. This protocol was approved by an Institutional Ethical Review Committee and all patients signed consent forms.

PBM remained positive for HIV-1 on co-culture in all placebo treated patients with the exception of one of the two samples from patients tested at week three. Placebo treatment did not affect HIV-1 infectivity of PBM. This result confirms other reports, which indicate that the virus infection detected in patients with antibody to HIV-1 usually persists over time. [See, e.g. J. A. Levy et al, Science, 225:840-842 (1984) and C. A. Andrews et al, J. Pediat., 111:672-677 (1987)].

In contrast, there was a steady decline in HIV-1 infectivity in PBM of thymopentin-treated patients with four of five patients (a response rate of 80%) having negative co-culture for HIV-1 at ten weeks.

The difference in incidence of virus infection at ten weeks between placebo and thymopentin treated patients was statistically significant ($p = 0.048$) when compared by a two-tailed Fisher exact test. This progression to co-culture negativity occurred progressively from weeks three to ten in the thymopentin-treated group. These results are graphically illustrated in the drawing.

Control of HIV proliferation and infectivity to such HIV viremic patients by the method described herein employing thymopentin could assist in the prevention of disease progression in the treated patient. Additionally, the treatment of such patients by the method described herein may reduce HIV transmission through contact by non-infected persons with the body fluids of the patient.

The above Example has been presented for illustrative purposes only. Numerous modifications and variations in the method are expected to occur to those of skill in the art. Such modifications are encompassed within the invention.

## Claims

1. The use of an active ingredient selected from thymopentin and analogs and derivatives thereof for preparation of a pharmaceutical composition suitable for reducing the infectivity of HIV virus in the peripheral blood cells and other body fluids of a viremic subject having peripheral blood mononuclear cells capable of transmitting the HIV virus to uninfected cells in co-culture.

2. The use according to claim 1 wherein said HIV virus is HIV-1 virus.

3. The use according to claim 1 or 2 wherein said viremic subject has no clinical symptons of HIV infection.

4. The use according to any of claims 1 to 3 wherein said pharmaceutical composition contains an amount of thymopentin between about 5 mg and about 500 mg.

5. The use according to claim 4 wherein said pharmaceutical composition is suitable for daily administration.

6. The use according to any of claims 1 to 5 wherein said pharmaceutical composition is in the form of unit dosages for administration between at least 1 and 3 times per week.

7. The use according to any of claims 1 to 6 wherein said active ingredient comprises a thymopentin analog capable of oral administration.

8. The use according to any of claims 1 to 6 wherein said pharmaceutical composition contains an amount of thymopentin effective to maintain a state of non-viremia or reduced viremia.

9. The use according to any of claims 1 to 8 wherein said pharmaceutical composition is suitable for parenteral administration.

10. The use according to any of claim 1 to 6 wherein said pharmaceutical composition contains about 50 mg thymopentin and is suitable for subcutaneous administration.

EP 0 335 726 A2